Europäisches Patentamt

(19) European Patent Office

Office européen des brevets

(11) Publication number: **0 444 883 A1**

## (12) EUROPEAN PATENT APPLICATION

(21) Application number: 91301551.7

(51) Int. Cl.⁵: **A61F 2/66**

(22) Date of filing: 26.02.91

(30) Priority: 28.02.90 GB 9004430
19.12.90 GB 9027531

(43) Date of publication of application:
04.09.91 Bulletin 91/36

(84) Designated Contracting States:
DE FR NL

(71) Applicant: CHAS. A. BLATCHFORD & SONS
LIMITED
Lister Road
Basingstoke Hampshire RG22 4AH (GB)

(72) Inventor: Harris, Graham James
61 Oakridge Road
Basingstoke, Hampshire (GB)
Inventor: Woolnough, Victor James
Lawrenny, Mary Lane
North Waltham, Hampshire (GB)

(74) Representative: Blatchford, William Michael et
al
Withers & Rogers 4 Dyer's Buildings Holborn
London EC1N 2JT (GB)

(54) An artificial foot.

(57)   An artificial energy-storing foot has a keel (12) having a lower spring portion (12B) connected at its posterior end as a cantilever to an upper mounting portion (12A). These two portions may be formed as single carbon fibre reinforced plastics moulding or individually as part of a two-piece keel with the ankle mounting portion rigid and the spring portion as a simple fibre reinforced strip. The resilience of the foot is adjustable by means of a removable resilient buffer element (27) mounted ahead of the ankle centre as a course adjustment device and, optionally, by means of a transversely extending support bar (28) which can be moved longitudinally behind the ankle centre by rotating an adjusting screw (30). Both the buffer (27) and the support bar (28) are mounted between the ankle mounting portion (12A) of the keel and the lower spring portion (12B). A resilient ankle joint (13) may be secured to the ankle mounting portion (12A), the joint comprising a ball and socket assembly with resilient elements (48, 54) arranged to allow plantar flexion of the joint but substantially to prevent dorsi-flexion.

EP 0 444 883 A1

44
42
50
52
54
46A
18A
12
32
12C
10A   30   28   14   26   12B  24   27A  27

13A
46
48
13
44A
56  16  12A
12F  12E
A
20
22
A
10
10B

12D

**Fig.1.**

54

56

**Fig.1B.**

27

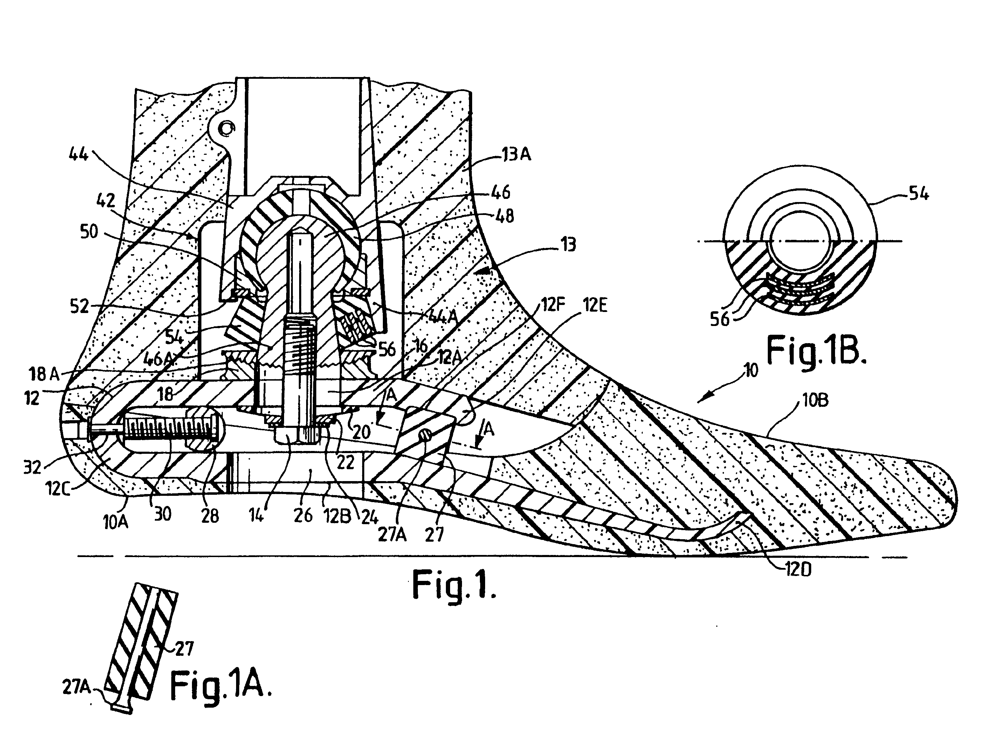

27A   **Fig.1A.**

# AN ARTIFICIAL FOOT

This invention relates to an artificial foot and to a lower limb prosthesis including such a foot.

It is well known that an artificial foot can be provided with an energy-storing resilient element to assist an amputee in obtaining a natural and comfortable gait and, in the case of the relatively active amputee, in running. It is also known from British Patent Application No. 2216423A to combine a foot having an energy-storing keel with a resilient ankle joint arranged to permit plantar, medial and lateral flexion about an ankle centre, but substantially to prevent dorsi-flexion. Such a combination is particularly suitable for obtaining a natural walking gait, but it has been found that the optimum keel resilience for walking is insufficiently stiff for patients capable of high activity levels.

According to one aspect of this invention, an energy-storing artificial foot comprises a keel having an upper load receiving portion, an elongate resiliently deflectable lower portion extending lengthwise in the foot from a heel portion of the foot to an anterior portion of the foot, the load receiving portion being connected to the lower keel portion in the heel portion of the foot, and characterised by a dorsi-flexion restricting device for location between an anterior end part of the load receiving portion and the lower keel portion.

In the preferred embodiment of the invention, the load receiving portion includes means for securing the foot to an upper limb component such as a shin component, and the dorsi-flexion restricting device is located at a position to the anterior of the securing means.

Restricting the dorsi-flexion of the keel in this way provides extra stiffness, which can improve certain patients' ability to run. The further in front of the shin axis or ankle joint centre the restricting device is placed, the greater is its effect in reducing dorsi-flexion of the keel. In one preferred embodiment of the invention, the restricting device is a buffer element in the form of a removable block of elastomeric material mounted between an anterior extension of the ankle mounting portion of the keel and the lower keel portion. Preferably, also, further means for adjusting the resistance of the keel to dorsi-flexion are provided in the form of a longitudinally adjustable support member located behind the shin axis or ankle centre and coupling a posterior part of the ankle mounting portion of the keel to the lower keel portion. Typically, the support member comprises a bar extending transversely of the foot between the ankle mounting portion of the keel and the lower keel portion, the bar being adjustably located by a longitudinally acting screw passing through an aperture in the said posterior connecting portion of the keel to be accessible at the rear of the foot. Such an assembly permits a wide range of keel stiffnesses to be obtained without altering the keel structure itself. Thus, the overall stiffness of the keel is affected both by the position of the longitudinally adjustable support member (as a fine adjustment) and the absence or presence of one of a series of elastomeric blocks of different hardnesses (as a coarse adjustment), thereby accommodating a wide range of patient weights and activity levels.

The foot is preferably incorporated in a limb prosthesis having an ankle joint assembly such as that disclosed in the above-mentioned British Patent Publication No. 2216423A, the content of which is incorporated in the present specification by reference. This assembly has a first part for connection to an upper component of the prosthesis, such as a shin component, a second part connected to the load receiving portion of the keel, and means connecting the second part to the first part and arranged to allow plantar flexion of the ankle joint assembly relative to a neutral state, but substantially to prevent dorsi-flexion.

The invention also includes, according to another aspect thereof, an energy storing artificial foot comprising a keel having an upper load receiving portion extending lengthwise in the foot in the anterior direction from the heel region of the foot to form a mounting part for mounting an upper limb component, and, secured to the upper load receiving portion in the heel region an elongate resiliently deflectable lower portion extending lengthwise in the foot from the said heel region to an anterior portion of the foot, the lower portion being spaced from the mounting part and being formed separately from the upper load receiving portion.

The resilient lower keel portion extends from the heel region at least into a metatarsal region of the foot, and is preferably of plastics material, particularly a fibre-reinforced plastics material. The lower keel portion, being formed separately from the upper keel portion, can be a component that is relatively easy to mould. For example, it may be formed as a single elongate strip or plate, preferably tapered in thickness from the heel region forwardly. The upper keel portion may be a substantially rigid component formed from, for example, fibre reinforced plastics or a metallic material such as aluminium alloy. The upper keel portion extends in an anterior-posterior direction, and may be generally in the form of a plate having a depending thickened posterior end part for housing a connecting element or elements securing the upper keel portion to the lower keel portion in the heel region of the foot. In this way, much of the resilience of a single U-shaped fibre-reinforced plastics keel is retained with a component which is simpler, and hence cheaper, to mould. This embodiment also offers the possibility of economical production of a range of keels of different stiffnesses for a given foot size. In its preferred form of a substantially rigid block, the upper keel portion allows a relatively high foot attachment bolt torque to be applied without damage to the keel, which is of advantage for active and heavy patients.

While the mounting part of the upper keel portion may be integral with the upper limb component (e.g. a shin component or part of a shin component) or ankle joint, the preferred embodiment of the invention has this mounting part provided with an interface for detachable mounting of the foot. This interface may be shaped to allow different heel height settings to be obtained. Typically, the mounting part mounts the upper limb component by having an oval aperture passing through the interface for housing a bolt threaded into the upper limb component.

The element for connecting the upper keel portion to the lower keel portion is preferably at least one bolt or rivet passing through the depending posterior end of the upper keel portion. In the case of a bolt, a threaded insert may be provided in the posterior end part of the lower keel portion.

A dorsi-flexion restricting device, particularly a resilient buffer element, may be fitted between an anterior end nose of the upper keel portion and the lower keel portion to modify the stiffness of the keel. A recess is provided in the lower face of the upper keel portion for locating this buffer element.

The foot may be connected to an ankle joint or directly to a shin member forming part of a limb prothesis and may be detachable from or integral with parts of the ankle joint and/or shin member.

The invention will now be described by way of example with reference to the drawings in which:-

Figure 1 is a vertical cross-section of a lower limb prosthesis in accordance with the invention, viewed from one side;

Figure 1A is a cross-section of a buffer element taken on a plane extending transversely of the prosthesis as shown by the line A-A in Figure 1;

Figure 1B is a partly sectioned plan view of a snubber ring forming part of an ankle joint of the prosthesis of Figure 1;

Figure 2 is a plan view of the foot shown in Figure 1;

Figure 3 is a side elevation of part of the prosthesis of Figure 1;

Figure 4 is a partly sectioned side elevation of an alternative artificial foot; and

Figure 5 is a plan view of a keel of the foot of Figure 4.

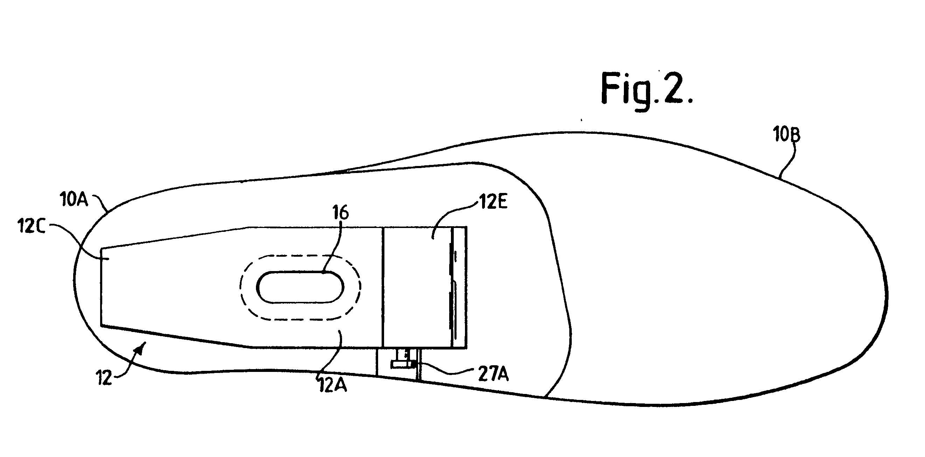

Referring to Figures 1 to 3, a lower limb prosthesis includes a foot 10 with an endoskeletal structure comprising a single-piece carbon-fibre reinforced plastics keel 12 having an upper plate portion 12A for mounting an ankle joint 13 for receiving loads through the ankle joint, and an integral elongate lower portion 12B in the form of a strip running lengthwise of the foot for transmitting the loads to the sole of the foot. The upper and lower keel portions 12A and 12B are joined together at their posterior ends adjacent the heel 10A of the foot by an integral curved connecting portion 12C so that the three portions 12A, 12B and 12C of the keel constitute a U-shaped section in side elevation, in the form of a folded strip.

The lower keel portion 12B is parallel-sided in its anterior region and terminates in the region of the ball of the foot in an upwardly directed lip 12D. Its thickness increases in the posterior direction, while the width narrows to a minimum in the connecting portion 12C. The upper plate portion 12A is also parallel-sided in its anterior region which terminates in an anterior end part 12E and is generally wider than the connecting portion. The lower keel portion 12B is seated in a moulded flexible polyurethane foam cosmesis 10B corresponding approximately in shape to the shape of the natural foot, while the ankle 13 is enclosed by the lower part of a shin cosmesis 13A. The two cosmesis parts 10B and 13A abut each other at a level below that of the upper plate portion 12A of the keel.

Attachment of the upper keel portion 12A to the ankle joint 13 is achieved by means of a vertical bolt 14 which passes through an oval aperture 16 in the upper portion 12A and is threaded in the joint 13 (Figure 1). Washers 18 and 20 are placed above and below the upper keel portion 12A to allow adjustment of the foot angle (in effect a heel height adjustment), the upper washer 18 having a serrated concave cylindrical surface 18A for mating with a corresponding convex surface on the ankle joint 13, and the lower washer 20 having a coaxial convex lower surface to allow proper seating of the head of the bolt 14 across the range of adjustment. Packing washers 22 and 24 are provided between the head of the bolt 14 and the convex washer 20. Access to the bolt head is gained via a second oval aperture 26 in the lower keel portion 12B and in the cosmesis 10B.

The stiffness of the keel 12 may be altered by inserting or removing, as required, a buffer element in the form of an elastomeric block 27 between the anterior end part 12E of the upper keel portion 12A and the lower keel portion 12D directly beneath.

A recess 12F is provided on the underside of the upper keel portion anterior end part 12E to house the buffer 27 when in place, and the buffer 27 itself has a transverse pin 27A which protrudes on the medial side of the foot as shown in Figure 1A to aid insertion and removal.

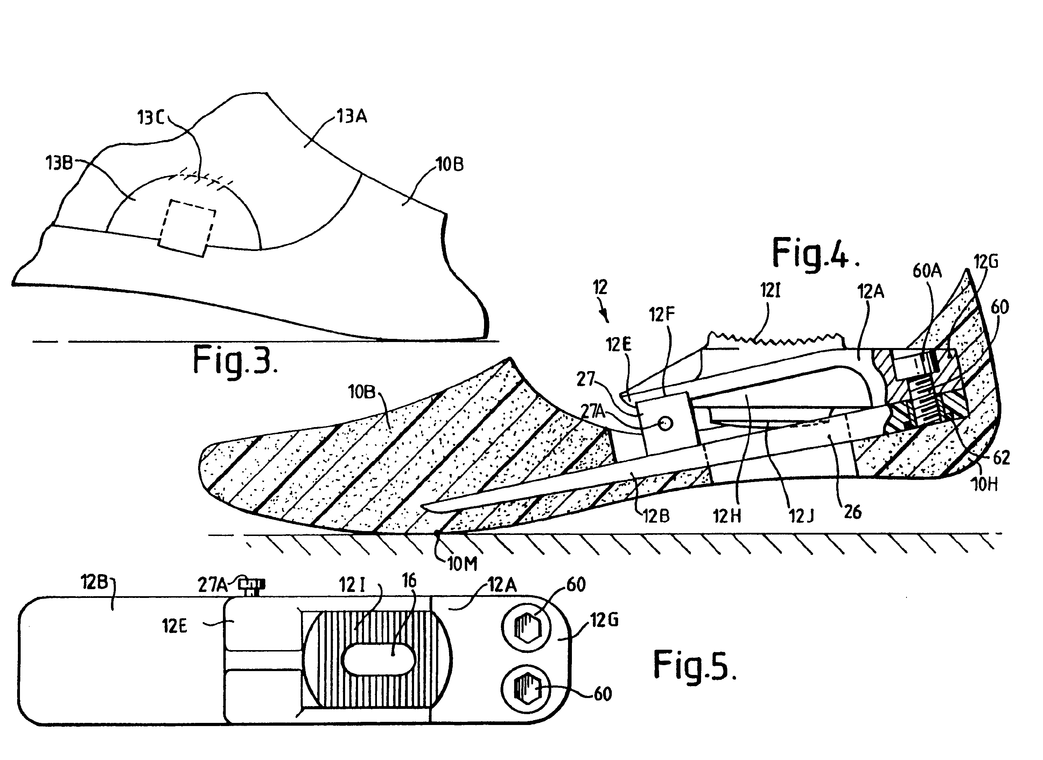

Referring to Figure 3, access to the buffer pin 27A is gained by lifting a hinged flap 13B of the shin cosmesis 13A, the flap being a displaceable cover hinged along its upper edge 13C as shown and extending to the lower edge of the shin cosmesis 13A where it meets the foot cosmesis 10B in order to cover an aperture in the cosmesis in registry with the buffer 27.

The degree to which the keel stiffness is increased by inserting the buffer element 27 depends on the hardness of the elastomeric material making up the block. It will be appreciated, then, that the keel stiffness can be varied in steps by providing blocks of different hardnesses.

Further variation in the stiffness of the keel 12, particularly when the buffer element 27 has been removed or is made of a very soft material, is achieved by means of a support member 28 (shown in figure 1) which couples the upper portion 12A to the lower portion 12B by engaging the lower and upper surfaces respectively of these members between the posterior connecting portion 12C and the bolt 14. The support member 28 comprises a transversely arranged aluminium alloy bar having a threaded central hole bored across it to receive a longitudinally positioned adjusting screw 30. The screw 30 passes through a bore 32 cut in the connecting portion 12C of the keel to emerge on the posterior face of the keel where it has a head 36 having a hexagon socket to suit an Allen key. The screw 30 is accessible at the heel of the foot where it can be rotated by a prosthetist or the patient.

It will be appreciated that the combination of the buffer element 27 and the adjustable support member 28 allows the stiffness of the keel to be adjusted over a wide range to suit the weight of the patient and his or her level of activity, thereby reducing the range of keels that need to be provided to suit patients of different sizes, weights, and activities. It is even possible for patients themselves to alter the keel stiffness temporarily to suit a particular activity. Such temporary alteration may be made without losing the setting produced by operating the adjusting control. Thus, for example, a keel stiffness for walking can be set by means of the screw adjuster, while a higher stiffness for running may be obtained if required by adding a removable dorsi-flexion restricting buffer element 27 which does not affect the screw adjuster.

To achieve good walking characteristics, the artificial foot 10 described above is combined with an ankle joint 13 allowing plantar ankle flexion and medial/lateral flexion but substantially preventing dorsi-flexion. Such a combination allows the benefits of ankle flexion to be retained together with the ability to store energy in the keel of the foot during the period immediately prior to "push-off" in the walking or running cycle.

Referring to Figures 1 and 1B, an example of an ankle joint 13 having the required flexion characteristics is in the form of a ball and socket joint 42. The joint 42 has a socket member formed by an upper limb component which, in this example, is the lower end portion 44 of a shin member. The socket member 44 houses a ball member 46 bolted to the upper portion 12A of the keel 12. A resilient part-spherical cover 48 is fitted over the ball member 46, the assembly of the ball member 46 and the cover 48 being clamped in the socket member 44 by an internal ring 50 held in position by an expanding circlip 52 housed in a groove in the shin member end portion 44.

The shin member end portion 44 has a depending skirt 44A extending around and spaced from a connection shank part 46A of the ball member 46. Between the skirt 44A and the shank 46A is a resilient snubber ring 54. The skirt 44A overlies only the medial, anterior and lateral outer faces of the snubber ring 54 thereby restricting medial, dorsi- and lateral flexion of the ankle joint, the plantar flexion allowed by the ball and socket joint being largely unaffected. To further restrict dorsi-flexion, the snubber ring 54 has encapsulated within its anterior portion a plurality of stiffening plates 56 which serve to restrict the resilience of the snubber ring in its anterior portion so as to increase further the resistance of the ankle joint to dorsi-flexion. Each plate 56 comprises an elongate rigid metal strip curved about an axis parallel to its surfaces and its end, and each is positioned transversely in the snubber ring 54 with its surfaces generally parallel to the outer part-conical surface of the ring 54. Restriction of dorsi-flexion is brought about not so much as a result of the presence of less rubber material in the anterior portion of the ring than in the medial or lateral portions, but rather by virtue of the limitation of the upward, downward and transverse deformation of the rubber material caused by the plates and their adhesion to the rubber material.

The ankle joint described above is a development of a joint disclosed in British Patent Specification No. 2161390A, the content of which is incorporated in this specification by reference. Its flexion characteristics are described and shown in the above mentioned British Patent Specification No. 2216423A.

Other means of restricting dorsi-flexion of the ankle joint may be employed. For instance, the plates 56 may be replaced by a single block of rigid material embedded in and/or bonded to the snubber ring.

Referring now to Figures 4 and 5 of the drawings, an alternative keel construction may be used in accordance with the invention. In this alternative construction, the keel 12 comprises a resilient lower keel portion 12L in the form an elongate plate moulded from carbon-fibre-reinforced plastics material. This plate 12L extends in an anterior-posterior direction in the foot from the heel region 10H of the foot to the metatarsal region 10M, tapering in thickness in the anterior direction with a more pronounced taper at the extreme anterior end.

Attached to the posterior end of the lower keel portion is an upper keel portion 12A which, in this embodiment, is a machined aluminum alloy plate which extends generally parallel to the lower keel portion 12B in the anterior-posterior direction, and which has a depending posterior end part 12G which abuts an upper surface of the posterior end part of the lower keel portion 12B. Two bolts 60 having their bolt heads 60A housed in the posterior end portion 12G of the upper keel portion 12A pass through bores in the posterior end part 12G and are threaded in inserts 62 embedded in the posterior end part of the lower keel portion 12B. It will be appreciated that other means for clamping the upper keel portion to the lower keel portion may be used, such as a clamping sleeve around the posterior end parts of both portions, or rivets in place of the bolts 60.

To add to the rigidity of the upper keel portion, one or more webs 12H are formed between the lower surface of the plate and the depending posterior end portion.

In order that the foot may be adjustably mounted on an upper limb component such as the ball and socket joint shown in Figure 1 the interface with the ankle joint, as before, comprises a raised mounting projection having a serrated concave cylindrical surface 12I. In the present embodiment, a boss 12J extends downwardly from the plate of the upper keel portion 12A in registry with the mounting interface 12L, the lower surface of the boss 12J being generally cylindrical and convex to receive the head of a bolt (not shown) passing through an oval slot 1216 passing through the upper keel portion 12A for fixing the upper keel portion 12U to the ankle joint. A slot 26 is provided in the lower keel portion to allow access to the bolt fixing the ankle joint and to provide a clearance for part of the boss 12J.

As in the embodiment of Figure 1, a rubber buffer 27 of selected hardness is fitted between an anterior nose 12E of the upper keel portion and the upper surface of the lower keel portion 12B, the buffer being removable so that the resilience of the keel can be modified according to the patient's requirements between a "soft" setting, in which the buffer 27 is removed and in which the complete length of the keel provides an energy-storing function, and a "hard" setting in which the buffer 27 acts as a deformable fulcrum. Evidently, buffers of different hardnesses can be used. Location of the buffer 27 is provided by a recess 12F in the downwardly directed surface of the anterior nose 12E, and insertion and removal is accomplished by means of a pin 27A or a similar projection forming part of the buffer 27, access being gained through a flap in the foot cosmesis as in the embodiment shown in Figures 1 to 5.

At least the lower keel portion 12B is encased in a foam cosmesis 10B in known manner, the cosmesis being shaped to resemble the natural foot.

While the foot particularly described here and shown in Figures 6 and 7 is intended for mounting to a resilient ankle joint, the upper keel portion may be integral with a shin component or may be bolted directly to a shin component, in which case it is preferable to provide a heel cushion of a different material from that of the cosmesis beneath the posterior end part of the lower keel portion 12B.

## Claims

1. An energy-storing artificial foot (10) comprising a keel (12) having an upper load receiving portion (12A), an elongate resiliently deflectable lower portion (12B) extending lengthwise in the foot from a heel portion (10A) of the foot to an anterior portion (10B) of the foot, the load receiving portion (12A) being connected to the lower keel portion (12B) in the heel portion (10A) of the foot, and characterised by a dorsi-flexion restricting device (27) for location between an anterior end part (12E) of the load receiving portion (12A) and the lower keel portion (12B).

2. An artificial foot according to claim 1, characterised in that the lower keel portion (12B) is separately formed from the load-receiving portion (12A), the lower keel portion being formed as a leaf spring which is secured at its posterior end to the load receiving portion, the latter being a substantially rigid component.

3. An artificial foot according to claim 1 or claim 2, characterised in that the load receiving portion (12A) includes means (14) for securing the foot (10) to an upper limb component, and wherein the restricting device (27) is located at a position to the anterior of the securing means (14).

4. An artificial foot according to claim 3, characterised in that the restricting device comprises a buffer element (27).

5. An artificial foot according to claim 4, characterised in that the buffer element (27) is displaceable from its position between the load receiving portion (12A) and lower keel portion (12B).

6. An artificial foot according to claim 4 or claim 5, characterised in that the buffer element (27) is a block of elastomeric material, and is insertable and removable into and from its location between the said anterior end part (12E) and the lower keel portion (12B) transversely of the foot (10).

7. An artificial foot according to claim 6, characterised by a cosmetic covering (13A) having a displaceable flap (13B) located on one side of the foot in registry with the buffer element (27).

8. An artificial foot according to any preceding claim, further characterised by an adjustable support member (28) coupling the upper load receiving portion (12A) and the lower keel portion (12B), the longitudinal position of the support member (28) being variable.

9. A lower limb prosthesis including a foot (10) according to any preceding claim and characterised by an ankle joint assembly (13) having a first part (44) for connection to an upper component of the prosthesis, a second part (46) connected to the load receiving portion (12A) of the keel (12), and means (44A, 48, 54, 56) connecting the second part (46) to the first part (44) and arranged to allow plantar flexion of the ankle joint assembly (13) relative to a neutral state, but substantially to prevent dorsi-flexion.

10. An energy-storing artificial foot comprising a keel (12) having an upper load receiving portion (12U) extending lengthwise in the foot (10) in the anterior direction from the heel region (10H) of the foot to form a mounting part for mounting an upper limb component, and, secured to the upper load receiving portion (12U) in the heel region (10H), an elongate resiliently deflectable lower portion(12L) extending lengthwise in the foot from the said heel region (10H) to an anterior portion (10B) of the foot, the lower portion (12L) being spaced from the mounting part and being formed separately from the upper load receiving portion (12U).

Fig.1.

Fig.1A.

Fig.1B.

EP 0 444 883 A1

Fig.2.

EP 0 444 883 A1

Fig.3.

Fig.4.

Fig.5.

EP 0 444 883 A1

European Patent Office

**EUROPEAN SEARCH REPORT**

Application Number

EP 91 30 1551

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y,D | EP-A-0 331 468 (BLATCHFORD)<br>* The entire document * | 1-4,8-10 | A 61 F 2/66 |
| Y | FR-A-2 138 082 (TRUMPLER)<br>* Page 7, line 29 - page 8, line 15; figures * | 1-4,8-10 | |
| A | | 6 | |
| A | GB-A- 105 293 (SALMON)<br>* Page 3, lines 4-6; figure 2 * | 1,4,6 | |
| A | GB-A-2 202 448 (PHILLIPS)<br>* Page 12, lines 6-18; page 15, lines 3-5; figures 5-11 * | 2,10 | |
| A | GB-A-2 070 439 (BLATCHFORD)<br>* Page 2, lines 73-76; page 3, lines 48-70; figures 1,4-6 * | 5,9 | |
| A | US-A-2 677 825 (RICHARDSON)<br>* Column 3, lines 24-29; figures 1,2,6 * | 7 | TECHNICAL FIELDS SEARCHED (Int. Cl.5) |
| A,D | GB-A-2 161 390 (BLATCHFORD)<br>* Abstract; figures * | 9 | A 61 F |
| A | CH-A- 92 523 (BUR) | | |
| A | GB-A- 120 445 (BLATCHFORD) | | |
| A | US-A-4 645 509 (POGGI) | | |

The present search report has been drawn up for all claims

| Place of search | Date of completion of the search | Examiner |
|---|---|---|
| THE HAGUE | 24-05-1991 | KLEIN C. |

EPO FORM 1503 03.82 (P0401)